# EUROPEAN PATENT SPECIFICATION

(11) **EP 0 623 309 B1**
(45) Date of publication and mention of the grant of the patent: **03.09.1997**
(21) Application number: 94850077.2
(22) Date of filing: 06.05.1994
(51) Int. Cl.: A61B 5/022

(54) **Sphygmomanometer cuff**
Manschette für Blutdruckmesser
Brassard pour sphygmomanomètre

(30) Priority: 06.05.1993 SE 9301553
(43) Date of publication of application: 09.11.1994
(73) Proprietor: POLYMER DIPPING CENTER AB, 262 72 Ängelholm (SE)
(72) Inventor: Atkins, Ian, S-269 00 Bastad (SE)
(74) Representative: Roth, Ernst Adolf Michael

(56) References cited:
- EP-A- 0 136 279
- GB-A- 2 253 789
- US-A- 4 210 154

## Description

This invention relates to a sphygmomanometer cuff comprising two or more separately inflatable, different-sized chambers, each having an air-supply duct.

A sphygmomanometer cuff usually consists of an inner flexible bladder with one or more connecting hoses, and a surrounding cover provided with Velcro™ fasteners or the like enabling the cuff to be fixed round an arm or a thigh when the blood pressure is to be measured. Normally, an air pump or the like and a manometer are also associated to the cuff.

The sphygmomanometer cuff most frequently used today has an inner bladder with a single inflatable chamber. To enable correct reading of the blood pressure, the user has to choose a cuff whose bladder is of the right size, i.e. a bladder of which above all the width is suited to the circumference of the arm or the thigh. According to European standards in course of preparation, the optimal dimensions of a bladder in a sphygmomanometer cuff are the following:
- Width:: 40% of the circumference
- Length:: 80-100% of the circumference.

The prior art encompasses a single-chamber sphygmomanometer cuff in which the inside of the cover is provided with marks relating to suitable cuff sizes. With the aid of these marks, the user is, when applying the cuff, able to establish whether the cuff has the right size or is too large or too small, in which case it has to be replaced with a cuff whose bladder is of another size. However, there is still a risk that the user will choose a wrong-sized cuff, despite the marks provided. One reason may be that the circumference of the arm or the thigh at issue is just outside the size interval for which the cuff is intended. Under these circumstances, the user may, e.g. if a cuff of the right size is not readily available or is being used by someone else, choose a cuff of the wrong size, resulting in incorrect reading of the blood pressure.

Furthermore, EP-B1-0 136 279 teaches a sphygmomanometer cuff of the type stated by way of introduction, which has several overlapping and different-sized chambers.

This EP patent also calls attention to the fact that the single-chamber cuff involves the risk of inaccurate measurements, which means that a patient may be given e.g. antihypertensive medicine by mistake. In the sphygmomanometer cuff according to EP-B1-0 136 279, one chamber of several chambers in the bladder is automatically selected. This is achieved by providing a branch system of hoses connected to the respective chambers, as well as a throttling means which is provided on the cuff and which, once the cuff has been applied in position for measuring the blood pressure, is automatically positioned so as to squeeze and block the hose or hoses leading to the chamber or chambers that are to remain inactive for the circumference of the arm or the thigh at issue.

However, the multi-chamber cuff according to EP-B1-0 136 279 suffers from the drawback of constraining the user to rely on the automatic selection of the chamber, as compared with the freedom offered by the single-chamber cuff. Moreover, the known multi-chamber cuff does not give the user any indication of which chamber is automatically selected, which not only entails an element of uncertainty when on the border between two chamber sizes, but also means that the size of the chamber actually used cannot be indicated on the patient's case record. As a result, one cannot with certainty compare a subsequent measurement by means of a similar cuff or a standard cuff with a measurement by means of the automatically adjustable cuff set forth in EP-B1-0 136 279. In addition, this known cuff is of comparatively complicated design.

US-A-4 210 154 discloses a sphygmomanometer cuff with two different-sized chambers having an air-supply duct and a selector valve which has two manually adjustable valve positions, corresponding to the chambers. The cuff in US-A-4 210 154 does not comprise any indicating means according to the present invention.

The object of the invention is to remedy the drawbacks of the prior-art multi-chamber sphygmomanometer cuff. In particular, the invention obviates the risk of incorrect readings when on the border between two size intervals.

According to the invention, this object is attained by a sphygmomanometer cuff comprising two or more separately inflatable, different-sized chambers, each having an air-supply duct, and a selector valve means which has a number of manually adjustable valve positions, corresponding in number to the chambers, and which has one side connected to the air-supply ducts of the chambers and another side connectible to an air pump, said cuff being characterised by indicating means which, when the sphygmomanometer cuff is applied in position round an arm or a thigh for measuring the blood pressure, give the user a visual indication, which depends on the circumference of the arm or the thigh, for manually adjusting the selector valve means to a valve position permitting selective inflation of a chamber of suitable size in view of the circumference of the arm or the thigh at issue.

With the inventive sphygmomanometer cuff, the user has perfect control over which size of the inflatable chamber is used in the measurement. Furthermore, the user will know if the measurement is carried out near the borderline between chamber sizes. This is a considerable advantage, e.g. when considering writing out a prescription for antihypertensive medicine. In such a borderline case, the user may himself choose the size of the chamber to be used. A possible course of action in such a borderline case would be to measure the blood pressure first with the chamber on one side of the border, and then with the chamber on the other side of the border, and subsequently determining the average value of the two measurements. The shift between the chambers is simple and expedient and does not in any way disturb the patient. When using different-sized single-chamber standard cuffs in such a borderline case, on the other hand, the whole cuff has to be replaced, which may worry or disturb the patient, which means that there is a risk of the blood pressure changing between the measurements.

Thus, the inventive sphygmomanometer cuff further permits the user to make a note of the size of the chamber or chambers used when measuring the blood pressure, enabling among other things a correct comparison with subsequent measurements, e.g. by means of standard cuffs of single-chamber type.

Moreover, the inventive sphygmomanometer cuff can be used along with a calibration method. In a first measurement, the blood pressure of the patient is measured intrally, to give a reliable value. Then, the blood pressure is measured by means of the inventive cuff, e.g. using two different-sized chambers. Then, the user is able to establish, with a high degree of certainty, which chamber gives the correct result for the specific patient, and no intral measurement is thus needed on future occasions.

It may be observed that, even if the automatically adjustable sphygmomanometer cuff according to EP-B1-0 136 270 were to be supplemented with means indicating which chamber has been automatically selected, the drawbacks of this known cuff would not be remedied, since the user would still be constrained to rely on the automatic selection of the chamber.

The invention has yet another advantage over the automatic cuff according to EP-B1-0 136 279. When applying a sphygmomanometer cuff, either the inventive cuff or the cuff according to EP-B1-0 136 279, there is always a risk that the cuff is wound too tightly or too loosely. The prior-art cuff does not give the user any warning of this at all, whereas the inventive cuff does so if the setting indication is on the border between two valve positions. The user may then take suitable steps: either measuring the blood pressure with two chambers or rearranging the cuff, optionally after measurement in the first position.

In a preferred embodiment of the inventive sphygmomanometer cuff, the indicating means comprise interval marks provided on the outside of the cuff and each corresponding to a chamber, as well as a pointer which projects from one longitudinal edge of the cuff and which, when the cuff is applied in position round an arm or a thigh for measuring the blood pressure, is located within an area where the cuff ends overlap and within a then visible interval mark corresponding to the circumference at issue. The pointer may essentially be an extension of the inner terminal edge of the cuff, i.e. the terminal edge first brought into contact with the arm or the thigh when the cuff is applied. The interval marks can be provided on a flap projecting from the longitudinal edge of the cuff, in which case the pointer projects farther from the longitudinal edge of the cuff than does the flap, such that the pointer projects behind the flap when the cuff is applied in position round an arm or a thigh. Also, the interval marks may consist of transverse lines on the outside of the cuff.

Moreover, the interval marks on the cuff may conveniently be supplemented with a written indication, or the like, urging the user to adjust the selector valve means to the correct position, e.g. of the type "SELECT 15 cm", "SELECT 12 cm", and so forth, indicating the width of the chamber to be chosen.

Preferably, the selector valve means has, for each of its valve positions, a position mark corresponding to the respective interval marks on the cuff, e.g. marks of the type "15", "12", and so forth.

In a special embodiment, enabling the user to spontaneously choose, when measurement is to be performed, the valve position corresponding to the indication given by the indicating means, the selector valve means may, apart from the above-mentioned valve positions enabling selective inflation of the corresponding chambers, have a neutral position preventing inflation of the chambers. The selector valve means may then be so designed as to always occupy this neutral position before measurement has begun. However, the user may, for one reason or another, choose another valve position than the one given by the indicating means.

It should be observed that the chambers in the inventive cuff preferably are formed of one and the same flexible bladder, as described in EP-B1-0 136 279, and that the cuff further has, in a manner known per se, a cover which surrounds the bladder and preferably is provided with the indicating means.

These and other distinctive features are recited in the appended claims and also appear from the following description of an illustrative embodiment of the inventive sphygmomanometer cuff, reference being made to the accompanying drawings, in which
Fig. 1 is a partly cut-away perspective view of a double-chamber bladder connected to a selector valve means for use in a sphygmomanometer cuff according to the invention
Fig. 2 is a perspective view of an inventive sphygmomanometer cuff comprising a bladder of the type shown in Fig. 1,
Fig. 3 is a perspective view illustrating the use of the sphygmomanometer cuff shown in Fig. 2, and
Fig. 4 is a schematic view showing an alternative embodiment of the selector valve means for use in a sphygmomanometer cuff according to the invention.

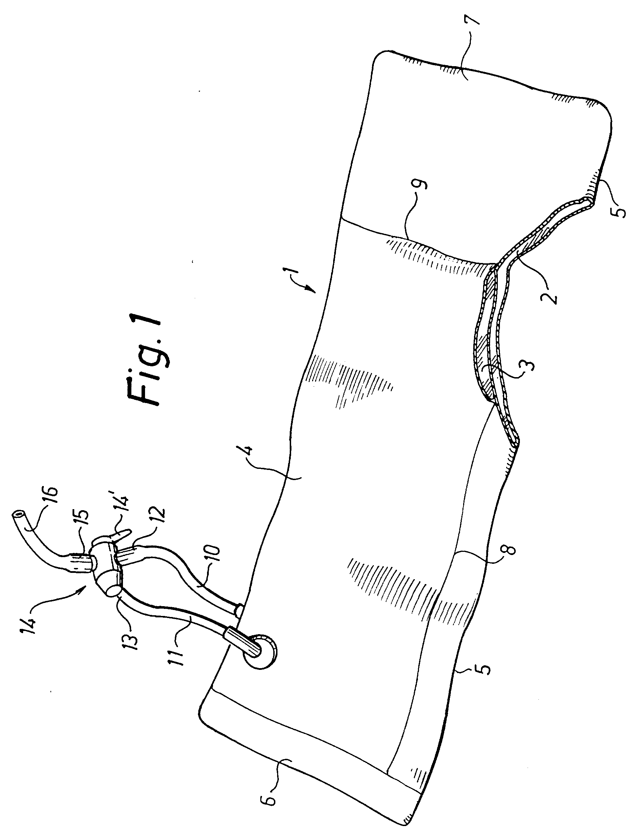

Fig. 1 shows a bladder, generally designated 1, which has but two different-sized chambers 2 and 3. However, it is to be understood that three or more different-sized chambers may, of course, be used, if required. The large chamber 2 is defined by two longitudinal edges 4 and 5 and two terminal edges 6 and 7, and the small chamber 3 is defined by two longitudinal edges 4 and 8 and two terminal edges 6 and 9. In the embodiment shown, the large chamber 2 has a width of about 15 cm, and the small chamber 3 has a width of about 12 cm.

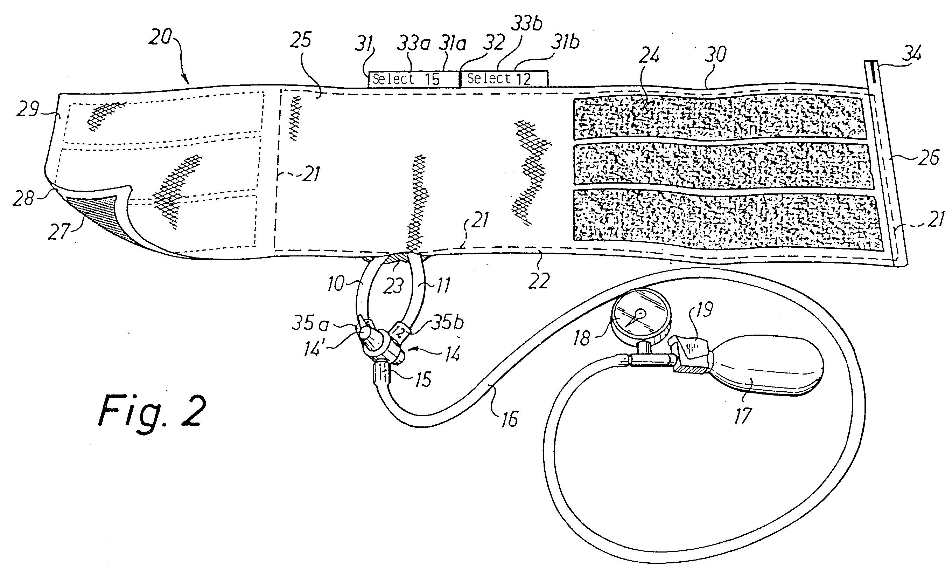

The chambers 2 and 3 each have an air-supply duct 10 and 11, respectively. At their distal ends, the ducts 10 and 11 are each connected to an outlet connection 12 and 13, respectively, of a selector valve means, generally designated 14. At an inlet connection 15, the selector valve means 14 is connected to a hose 16, whose other end is connected to an air pump 17 via a manometer 18 and a manually adjustable non-return valve 19, as illustrated in Fig. 2.

The flexible bladder 1 is arranged inside a rectangular cover 20 of some suitable material. In Fig. 2, the dashed line 21 marks the outer boundary of the bladder 1. At one longitudinal edge 22, the cover 20 has a lateral opening 23 through which the air-supply ducts 10 and 11 pass to the selector valve means 14. Furthermore, the cover 20 has, in a manner known per se, fastening means in the form of Velcro™ fasteners arranged at 24 on the outside 25 of the cover 20 by the inner edge 26 of the cover (i.e. the edge located closest to the arm or the thigh when the cuff is applied in position for measuring the blood pressure), and at 27 on the inside 28 of the cover by the outer terminal edge 29 of the cover.

A flap 31, which is provided with two interval marks 31a and 31b in the form of two boxes separated by a marking line 32, projects from the longitudinal edge 30 that is facing away from the ducts 10 and 11. The boxes 31a and 31b are provided with, respectively, a written indication 33a "Select 15" and a written indication 33b "Select 12". A pointer or flag 34 is also provided at the same longitudinal edge 30 by the inner terminal edge 26 of the cover 20 and projects farther from the longitudinal edge 30 than does the flap 31.

As shown in Fig. 2, the outlet connections 12 and 13 of the selector valve means 14 each have a visible position mark "15" and "12" respectively corresponding to the indications "Select 15" and "Select 12" provided on the flap 31.

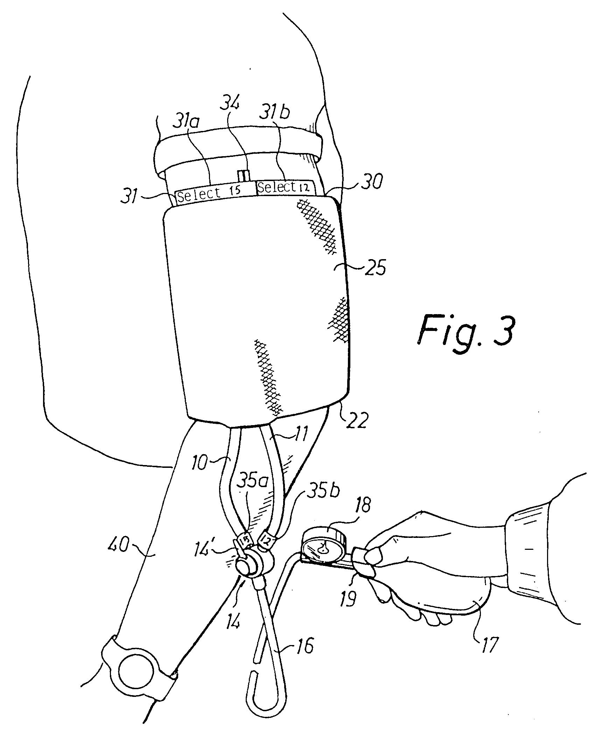

The use of the above sphygmomanometer cuff according to the invention will now be described in more detail with reference to Fig. 3, showing the cuff when applied in position round an arm 40 for measuring the blood pressure. Thus, the cover 20 of the cuff has been wound round the arm 40 in known manner and is fixed by the Velcro™ fasteners 24 and 27 in overlapping relationship.

When the cuff is thus applied in position, the indicating means, i.e. the flap 31 and the pointer 34, are both visible and, as appears from the Figure, give the user a visual indication, depending on the circumference of the arm, for manually adjusting the selector valve means 14 to the valve position (here "15") permitting selective inflation of the chamber (here the large chamber 2) that has a suitable size in view of the circumference of the arm. As appears from the Figure, the pointer 34 projects behind the flap 31.

In the situation illustrated in Fig. 3, the pointer 34 is relatively close to the borderline 32 between the two interval marks. This information, which is immediately available to the user, i.e. that measurement is carried out in a borderline position, heightens awareness that the measurement results should be checked with special care, e.g. by performing a measurement also with the small chamber 3 in position "12".

In the embodiment shown, the selector valve means 14 has a manually adjustable valve member which, by means of a pin 14', clearly indicates the current valve position, i.e. which chamber is to be inflated by the air pump 17.

It may be advantageous not to have ducts 10 and 11 of excessive length, so that the user has both the position of the pointer 34 on the flap 31 and the current position of the selector valve means 14 in his field of vision.

The selector valve means 14 may be designed in many different ways, depending on the number of chambers and on the connection of the air pump and the manometer, as well as on whether there are to be other valve positions than those directly corresponding to the interval marks on the flap 31.

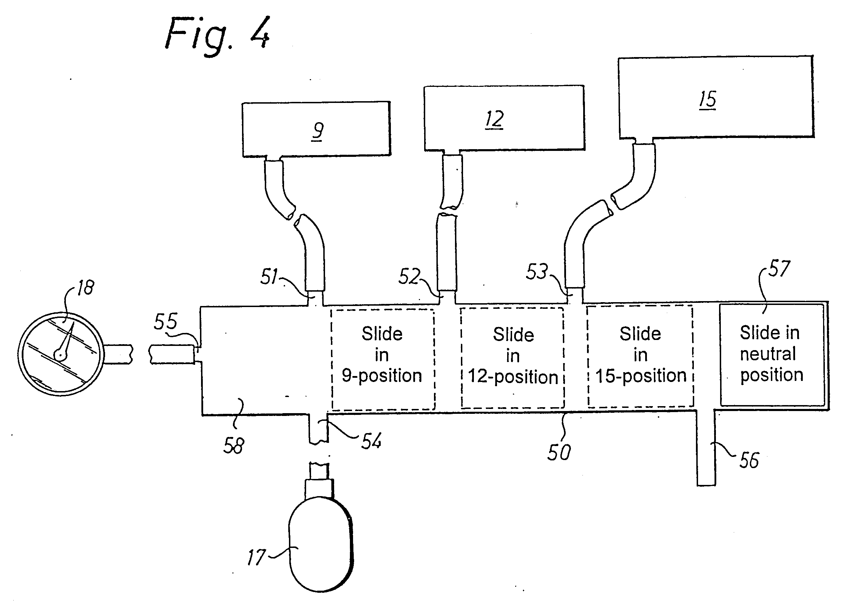

Fig. 4 schematically illustrates an alternative embodiment of the selector valve means. The valve shown in Fig. 4 is intended for a three-chamber cuff, whose chambers have a width of 9 cm, 12 cm and 15 cm, respectively. In Fig. 4, the chambers are shown as separate units, but are, in actual practice, preferably arranged in overlapping relationship in the form of a continuous bladder, in accordance with the embodiment illustrated in Figs 1-3.

The valve comprises a valve housing 50 having three outlet connections 51, 52 and 53 connected to the respective chambers, one inlet connection 54 connected to the air pump 17, one connection 55 connected to the manometer 18, and one connection 56 or opening for the discharge of air. Furthermore, the valve includes a manually displaceable valve member or slide 57 by means of which the user may choose which of the three chambers is to be inflated. If the smallest "9"-chamber is to be used, the valve slide 57 is set in the left-hand position of the three positions marked by dashed lines, in which case the connection 51 is open and the connections 52 and 53 are blocked. If use instead is to be made of the "12"-chamber, the valve slide 57 is set in the central position of the three positions marked by dashed lines in Fig. 4, in which case the connections 51 and 52 are open and the connection 53 is blocked. The "12"-chamber being larger than the "9"-chamber, only the former will be inflated at this point. Finally, if use is to be made of the "15"-chamber, all three connections 51-53 are opened.

Regardless of which chamber is being used, both the air pump 17 and the manometer 18 are in communication with the common compartment 58 in the valve housing 50 to enable correct reading of the pressure.

To enable use of the valve in Fig. 4 also in the event of the manometer and the air pump being integrally formed, as in Figs 2 and 3, the connection 55 may optionally be designed as an initially closed but rupturable opening.

Apart from the three positions marked by dashed lines, the valve slide 57 can also be set in a neutral position, which is indicated by full lines farthest to the right in the valve housing 50. In this neutral position, the air-discharge opening 56 is open so as to empty the cuff after use, and all inflation of the cuff is prevented. With such a construction, the user has to choose one of the three positions of use deliberately, which reduces the risk of the wrong chamber being chosen.

The invention can be modified in many ways within the scope of the appended claims. For instance, the flap can be dispensed with, to be replaced with transverse lines or the like provided directly on the outside 25 of the cover 20. Furthermore, the pointer 34 may be otherwise designed and be placed elsewhere than at the inner edge 26. Also, the cover 20 and the bladder 1 may be designed as a single unit.

Moreover, the chambers may each be formed of a separate bladder. A possible variant, used instead of the illustrated embodiment with overlapping chambers, is to arrange an inner rectangular chamber as well as one or more outer chambers each provided as a "frame" completely outside the surface of the inner chamber, optionally only at one short side and one long side of the inner chamber. For instance, provision may be made of an inner chamber having a width of 12 cm and an outer frame which, on the long side of the inner chamber, has a width of 3 cm, such that simultaneous inflation of both chambers has the same effect as in the case of a 15-cm-broad chamber of greater length than the 12-cm-broad chamber.

## Claims

1. A sphygmomanometer cuff comprising two or more separately inflatable, different-sized chambers (2, 3), each having an air-supply duct (10, 11), and a selector valve means (14), which has a number of manually adjustable valve positions, corresponding in number to the chambers, and which has one side (12, 13) connected to the air-supply ducts (10, 11) of the chambers (2, 3) and another side (15) connectible to an air pump (17), **characterised by**
indicating means (31-34) which, when the sphygmomanometer cuff is applied in position round an arm (40) or a thigh for measuring the blood pressure, give the user a visual indication, which depends on the circumference of the arm or the thigh, for manually adjusting the selector valve means (14) to a valve position permitting selective inflation of a chamber of suitable size in view of the circumference of the arm or the thigh at issue.

2. The cuff of claim 1, **characterised** in that the indicating means (31-34) comprise interval marks (31a, 33a; 31b, 33b provided on the outside (25) of the cuff and each corresponding to a chamber (2, 3), as well as a pointer (34) which projects from one longitudinal edge (30) of the cuff and which, when the cuff is applied in position round an arm (40) or a thigh for measuring the blood pressure, is located within an area where the cuff ends (26, 29) overlap and within a then visible interval mark (31a, 33a; 31b, 33b) corresponding to the circumference at issue.

3. The cuff of claim 2, **characterised** in that the pointer (34) essentially is an extension of the inner terminal edge (26) of the cuff.

4. The cuff of claim 2 or 3, **characterised** in that the selector valve means (14) for each of its valve positions is provided with a position mark (35a, 35b) corresponding to the respective interval marks (31a, 33a; 31b, 33b) on the cuff.

5. The cuff of any one of claims 2-4, **characterised** in that the interval marks (31a, 33a; 31b, 33b) are arranged on a flap (31) projecting from the longitudinal edge (30) of the cuff, and that the pointer (34) projects farther from the longitudinal edge (30) of the cuff than does the flap (31), such that the pointer (34) projects behind the flap (31) when the cuff is applied in position round an arm or a thigh.

6. The cuff of any one of the preceding claims, **characterised** in that the selector valve means (14), in addition to the valve positions enabling selective inflation of the corresponding chambers, has a neutral position preventing inflation of the chambers.

7. The cuff of claim 6, **characterised** in that the selector valve means (14) is adapted to occupy the neutral position when air is to be discharged from the cuff after use.

8. The cuff of any one of the preceding claims, **characterised** in that the selector valve means (14) has a collecting chamber (58) communicating with the air pump (17), and a valve member (57) which, depending on the valve position set, opens or blocks the passage between the collecting chamber (58) and the air-supply ducts (51-53) of the chambers.

9. The cuff of claim 8, **characterised** in that the selector valve means (14) has a connection (55) opening in the collecting chamber 58 and adapted to be connected to a manometer (18).

10. The cuff of any one of the preceding claims, **characterised** in that the chambers (2, 3) are formed of one and the same flexible bladder (1), and that it further has a cover (20) which surrounds the bladder (1) and on which the indicating means (31a, 33a; 31b, 33b) are arranged.

## Patentansprüche

1. Blutdruckmeßgerät-Manschette, die zwei oder mehr separat aufblasbare Kammern unterschiedlicher Größe (2, 3) umfaßt, von denen jede über eine Luftzufuhr-Leitung (10, 11) verfügt, und eine Wahlventil-Vorrichtung (14), welche eine Anzahl von manuell einstellbaren Ventilpositionen aufweist, die in ihrer Anzahl der Anzahl der Kammern entsprechen, und bei denen eine Seite (12, 13) mit den Luftzufuhr-Leitungen (10, 11) der Kammern (2, 3) verbunden ist sowie eine andere Seite (15), die an eine Luftpumpe (17) anschließbar ist, gekennzeichnet durch
Anzeigevorrichtungen (31 - 34), die, wenn die Blutdruckmeßgerät-Manschette um einen Arm (40) oder einen Oberschenkel herum zur Messung des Blutdruckes angelegt wird, dem Nutzer eine visuelle Anzeige geben, die vom Umfang des Armes oder des Oberschenkels abhängt, damit die Wahlventil-Vorrichtung (14) manuell auf eine Ventilposition eingestellt werden kann, die ein selektives Aufblasen einer Kammer geeigneter Größe im Verhältnis zum Umfang des betreffenden Armes oder Oberschenkels gestattet.

2. Manschette nach Anspruch 1, dadurch gekennzeichnet, daß die Anzeigevorrichtungen (31 - 34) Abstandsmarken (31a, 33a; 31b, 33b) auf der Außenseite der Manschette umfassen, die jeweils einer Kammer (2, 3) entsprechen, sowie einen Anzeiger (34), der aus einer Längskante (30) der Manschette hervorsteht und welcher, wenn die Manschette rund um einen Arm (40) oder einen Oberschenkel für die Messung des Blutdruckes angelegt wird, sich innerhalb einer Fläche befindet, wo die Manschettenenden (26, 29) überlappen und innerhalb einer dann sichtbaren Abstandsmarke (31a, 33a; 31b, 33b), die dem betreffenden Umfang entspricht.

3. Manschette nach Anspruch 2, dadurch gekennzeichnet, daß der Anzeiger (34) im wesentlichen eine Verlängerung der inneren Abschlußkante (26) der Manschette ist.

4. Manschette nach Anspruch 2 oder 3, dadurch gekennzeichnet, daß die Wahlventil-Vorrichtung (14) für jede ihrer Ventilpositionen eine Positionsmarke (35a, 35b) aufweist, die den entsprechenden Abstandsmarken (31a, 33a; 31 b, 33b) auf der Manschette entspricht.

5. Manschette nach einem der Ansprüche 2 - 4, dadurch gekennzeichnet, daß die Abstandsmarken (31a, 33a; 31b, 33b) an einer Klappe (31) angeordnet sind, die aus der Längskante (30) der Manschette hervorsteht, und daß der Anzeiger (34) weiter aus der Längskante (30) der Manschette hervorsteht als die Klappe (31), dergestalt, daß der Anzeiger (34) hinter der Klappe (31) hervorsteht, wenn die Manschette rund um einen Arm oder einen Oberschenkel angebracht wird.

6. Manschette nach einem der vorhergehenden Ansprüche, dadurch gekennzeichnet, daß die Wahlventil-Vorrichtung (14) zusätzlich zu den Ventilpositionen, die ein selektives Aufblasen der entsprechenden Kammern ermöglichen, eine neutrale Position hat, die das Aufblasen der Kammern verhindert.

7. Manschette nach Anspruch 6, dadurch gekennzeichnet, daß die Wahlventil-Vorrichtung (14) so eingestellt wird, daß sie die neutrale Position einnimmt, wenn nach der Verwendung Luft aus der Manschette abgelassen werden soll.

8. Manschette nach einem der vorhergehenden Ansprüche, dadurch gekennzeichnet, daß die Wahlventil-Vorrichtung (14) eine Sammelkammer (58) hat, die mit der Luftpumpe (17) in Verbindung steht, sowie ein Ventilelement (57), welches, abhängig von der eingestellten Ventilposition, den Durchlaß zwischen der Sammelkammer (58) und den Luftzufuhr-Leitungen (51 - 53) der Kammern öffnet oder blockiert.

9. Manschette nach Anspruch 8, dadurch gekennzeichnet, daß die Wahlventil-Vorrichtung (14) eine Anschlußöffnung (55) in die Sammelkammer (58) hat, ausgelegt für den Anschluß an einen Druckmesser (18).

10. Manschette nach einem der vorhergehenden Ansprüche, dadurch gekennzeichnet, daß die Kammern (2, 3) aus ein und derselben flexiblen Blase (1) gebildet werden, und daß sie weiterhin einen Überzug (20) hat, welcher die Blase (1) umgibt, und auf welchem die Anzeigevorrichtungen (31a, 33a; 31b, 33b) angeordnet sind.

## Revendications

1. Brassard pour sphygmomanomètre comprenant deux ou plus de deux chambres (2, 3) de tailles différentes, gonflables séparément, dotées chacune d'un tuyau d'alimentation en air (10, 11), et une vanne de sélection (14) ayant un nombre de positions de vanne ajustables manuellement et correspondant au nombre de chambres, et dont l'un des côtés (12, 13) est relié aux tuyaux d'alimentation en air (10, 11) des chambres (2, 3), l'autre côté (15) pouvant être relié à une pompe à air (17), caractérisé en ce qu'il comporte
des moyens indicateurs (31 à 34) qui, lorsque le brassard pour sphygmomanomètre est mis en position autour d'un bras (40) ou d'une cuisse pour mesurer la tension artérielle, fournissent à l'utilisateur une indication visuelle qui dépend de la circonférence du bras ou de la cuisse, pour ajuster manuellement la vanne de sélection (14) sur la position de vanne permettant le gonflage sélectif d'une chambre de taille appropriée, correspondant à la circonférence du bras ou de la cuisse en question.

2. Brassard selon la revendication 1, caractérisé en ce que les moyens indicateurs (31 à 34) sont constitués par des marques d'intervalle (31a, 33a ; 31b, 33b) disposées à l'extérieur (25) du brassard, chacune d'elles correspondant à une chambre (2, 3), ainsi que par un index (34) qui dépasse de l'un des bords longitudinaux (30) du brassard et qui, lorsque le brassard est mis en position autour d'un bras (40) ou d'une cuisse pour mesurer la tension artérielle, se trouve dans une zone où les extrémités (26, 29) du brassard se recouvrent et à l'intérieur d'une marque d'intervalle (31a, 33a ; 31b, 33b) alors visible et qui correspond à la circonférence en question.

3. Brassard selon la revendication 2, caractérisé en ce que l'index (34) est essentiellement une extension du bord d'extrémité intérieur (26) du brassard.

4. Brassard selon l'une ou l'autre des revendications 2 et 3, caractérisé en ce que la vanne de sélection (14) comporte, pour chacune de ses positions de vanne, un repère de position (35a, 35b) correspondant à la marque d'intervalle respective (31a, 33a ; 31b, 33b) du brassard.

5. Brassard selon l'une quelconque des revendications 2 à 4, caractérisé en ce que les marques d'intervalle (31a, 33a ; 31b, 33b) sont disposées sur une patte (31) dépassant du bord longitudinal (30) du brassard, et en ce que l'index (34) dépasse un peu plus du bord longitudinal (30) du brassard que ne le fait la patte (31), de telle sorte que l'index (34) dépasse derrière la patte (31) lorsque le brassard est mis en position autour d'un bras ou d'une cuisse.

6. Brassard selon l'une quelconque des revendications précédentes, caractérisé en ce que la vanne de sélection (14) comporte, en plus des positions de vanne permettant de choisir le gonflage des chambres correspondantes, une position neutre empêchant le gonflage des chambres.

7. Brassard selon la revendication 6, caractérisé en ce que la vanne de sélection (14) est prévue pour être en position neutre lorsque l'air doit être évacué du brassard après son utilisation.

8. Brassard selon l'une quelconque des revendications précédentes, caractérisé en ce que la vanne de sélection (14) comporte une chambre collectrice (58) communiquant avec la pompe à air (17), ainsi qu'un élément de vanne (57) qui, en fonction de la position choisie pour la vanne, ouvre ou ferme le passage entre la chambre collectrice (58) et les tuyaux d'alimentation en air (51 à 53) des chambres.

9. Brassard selon la revendication 8, caractérisé en ce que la vanne de sélection (14) comporte un raccord (55) ouvrant sur la chambre collectrice (58) et prévu pour être relié à un manomètre (18).

10. Brassard selon l'une quelconque des revendications précédentes, caractérisé en ce que les chambres (2, 3) sont formées dans une seule et même poche élastique (1) et en ce qu'il comporte en outre une enveloppe (20) qui entoure la poche (1) et sur laquelle sont disposés les moyens indicateurs (31a, 33a ; 31b, 33b).
